# EUROPEAN PATENT SPECIFICATION

(11) **EP 1 755 666 B1**
(45) Date of publication and mention of the grant of the patent: **15.12.2010**
(21) Application number: 05747432.2
(22) Date of filing: 26.05.2005
(51) Int. Cl.: A61K 39/145, A61K 9/127, A61K 47/46, A61K 47/48, A61K 39/39

(54) **VACCINE COMPOSITIONS COMPRISING VIROSOMES AND A SAPONIN ADJUVANT**
IMPFSTOFFZUSAMMENSETZUNGEN MIT VIROSOMEN UND EINEM SAPONIN-ADJUVANS
COMPOSITIONS DE VACCIN COMPRENANT DES VIROSOMES ET UN ADJUVANT A BASE DE SAPONINE

(30) Priority: 28.05.2004 GB 0412039; 02.06.2004 GB 0412304
(43) Date of publication of application: 28.02.2007
(60) Divisional application: 10178216.7
(73) Proprietor: GlaxoSmithKline Biologicals SA, 1330 Rixensart (BE)
(72) Inventor: COLLER, Beth-Ann, B-1330 Rixensart (BE); HENDERICKX, Véronique, B-1330 Rixensart (BE); GARCON, Nathalie M.J., B-1330 Rixensart (BE)
(74) Representative: Crepin, Carine Marie Blanche
(86) International application number: PCT/EP2005/005786
(87) International publication number: WO 2005/117958

(56) References cited:
- WO-A-2004/016281
- SJOLANDER A ET AL: "Kinetics, localization and cytokine profile of T cell responses to immune stimulating complexes (iscoms) containing human influenza virus envelope glycoproteins" VACCINE, BUTTERWORTH SCIENTIFIC. GUILDFORD, GB, vol. 15, no. 9, June 1997 (1997-06), pages 1030-1038, XP004115372 ISSN: 0264-410X
- MISCHLER R ET AL: "Inflexal<(>R)V a trivalent virosome subunit influenza vaccine: production" VACCINE, BUTTERWORTH SCIENTIFIC. GUILDFORD, GB, vol. 20, 20 December 2002 (2002-12-20), pages B17-B23, XP004397481 ISSN: 0264-410X
- CHEN D ET AL: "Epidermal powder immunization of mice and monkeys with an influenza vaccine" VACCINE, BUTTERWORTH SCIENTIFIC. GUILDFORD, GB, vol. 21, no. 21-22, 20 June 2003 (2003-06-20), pages 2830-2836, XP004429681 ISSN: 0264-410X
- STANLEY P ET AL: "THE POLY PEPTIDES OF INFLUENZA VIRUS PART 8 LARGE-SCALE PURIFICATION OF THE HEM AGGLUTININ" VIROLOGY, vol. 56, no. 2, 1973, pages 640-645, XP002349822 ISSN: 0042-6822
- MARKGRAF K ET AL: "Lipid composition of virosomes modulates their fusion efficiency with cryopreserved bull sperm cells" CLONING, MARY ANN LIEBERT, NEW YORK, NY, US, vol. 3, no. 1, 2001, pages 11-21, XP009016489 ISSN: 0737-1454
- MASTROBATTISTA E ET AL.: "Preparation of influenza virosomes by post-insertion of influenza virus spike proteins into preformed liposomes"[Online] 7 September 2001 (2001-09-07), pages 122-140, XP002349823 Retrieved from the Internet: URL:http://igitur-archive.library.uu.nl/di ssertations/1985758/c4.pdf> [retrieved on 2005-10-18]
- KENNEY RICHARD T ET AL: "Survey of human-use adjuvants." EXPERT REVIEW OF VACCINES. APR 2003, vol. 2, no. 2, April 2003 (2003-04), pages 167-188, XP008054103 ISSN: 1476-0584
- COPLAND M J ET AL: "Hydration of lipid films with an aqueous solution of Quil A: a simple method for the preparation of immune-stimulating complexes." INTERNATIONAL JOURNAL OF PHARMACEUTICS. 10 MAR 2000, vol. 196, no. 2, 10 March 2000 (2000-03-10), pages 135-139, XP002349821 ISSN: 0378-5173
- KERSTEN G F A ET AL: "Liposomes and ISCOMs" VACCINE, BUTTERWORTH SCIENTIFIC. GUILDFORD, GB, vol. 21, no. 9-10, 14 February 2003 (2003-02-14), pages 915-920, XP004402617 ISSN: 0264-410X
- HOMHUAN ATTHACHAI ET AL: "Virosome and ISCOM vaccines against Newcastle disease: Preparation, characterization and immunogenicity" EUROPEAN JOURNAL OF PHARMACEUTICAL SCIENCES, vol. 22, no. 5, August 2004 (2004-08), pages 459-468, XP002349824 ISSN: 0928-0987
- HUCKRIEDE ANKE ET AL: "The virosome concept for influenza vaccines." VACCINE. 8 JUL 2005, vol. 23 Suppl 1, 23 May 2005 (2005-05-23), pages S26-S38, XP004986025 ISSN: 0264-410X

### SEQUENCE LISTING

<110> Henderickx, Veronique
   Garcon, Nathalie
   Coller Beth-Ann
<120> Vaccine
<130> VB60863
<160> 5
<170> FastSEQ for Windows Version 3.0
<210> 1
   <211> 20
   <212> DNA
   <213> Human
<400> 1
   tccatgacgt tcctgacgtt 20
<210> 2
   <211> 18
   <212> DNA
   <213> Human
<400> 2
   tctcccagcg tgcgccat 18
<210> 3
   <211> 30
   <212> DNA
   <213> Human
<400> 3
   accgatgacg tcgccggtga cggcaccacg 30
<210> 4
   <211> 24
   <212> DNA
   <213> Human
<400> 4
   tcgtcgtttt gtcgttttgt cgtt 24
<210> 5
   <211> 20
   <212> DNA
   <213> Human
<400> 5
   tccatgacgt tcctgatgct 20

## Description

### TECHNICAL FIELD

The present invention relates to compositions containing influenza virosomes, in particular adjuvanted virosomes, to methods for preparing them and to their use in prophylaxis or therapy. In particular the present invention relates to compositions comprising an influenza, virosome preparation, and QS21.

### TECHNICAL BACKGROUND

An enveloped virus is one in which the virus core is surrounded by a lipid-rich outer coat containing viral proteins. Amongst such viruses are virus from the the following families: flaviviridae (i.e. *dengue* virus, *Hepatitis* C virus HEV, *Japanese encephalitis* virus, *Yellow fever* virus, West Nile virus), Poxviridae (i.e. Cowpox virus, Monkeypox virus, vaccinia virus, *Variola virus),* Retroviridae (i.e. Immuno deficiency viruses *HIVI*SIV, paramyxoviridae (i.e. *Measles* virus, *Mumps* virus, *Parainfluenza* viruses, metapneumovirus, Respiratory Syncytial virus *RSV*), and Orthomyxoviridae (i.e. *influenza viruses*) for example.

Human respiratory syncytial virus (RSV) is a member of the Paramyxoviridiae family of viruses and causes lower respiratory tract illness, particularly in young children and babies. Recent reports suggest that RSV is also an important pathogen in adults, particularly the elderly.

RSV is an enveloped virus with a non-segmented, negative strand ribonucleic acid (RNA) genome of 15,222 nucleotides that codes for 11 messenger RNAs, each coding for a single polypeptide. Three of the eleven proteins are transmembrane surface proteins: the G (attachment), F (fusion) and SH proteins. One protein is the virion matrix protein (M), three proteins are components of the nucleocapsid (N, P and L), and 2 proteins are nonstructural (NS1 and NS2). There are two further proteins M2-1 and M2-2. Two antigenically distinct sub-groups of RSV exist, designated subgroups A and B. Characterisation of strains from these sub-groups has determined that the major differences reside on the G proteins, while the F proteins are conserved.

Influenza virus is one of the most ubiquitous viruses present in the world, affecting both humans and livestock. The economic impact of influenza is significant.

The influenza virus is an RNA enveloped virus with a particle size of about 125 nm in diameter. It consists basically of an internal nucleocapsid or core of ribonucleic acid (RNA) associated with nucleoprotein, surrounded by a viral envelope with a lipid bilayer structure and external glycoproteins. The inner layer of the viral envelope is composed predominantly of matrix proteins and the outer layer mostly of the host-derived lipid material. The surface glycoproteins neuraminidase (NA) and haemagglutinin (HA) appear as spikes, 10 to 12 nm long, at the surface of the particles. It is these surface proteins, particularly the haemagglutinin, that determine the antigenic specificity of the influenza subtypes.

Typical influenza epidemics cause increases in incidence of pneumonia and lower respiratory disease as witnessed by increased rates of hospitalisation or mortality. The elderly or those with underlying chronic diseases are most likely to experience such complications, but young infants also may suffer severe disease. These groups in particular therefore need to be protected.

Influenza vaccines of all kinds are classically trivalent and can take the form of either live attenuated vaccines or inactivated formulations. They usually contain antigens derived from two influenza A virus strains and one influenza B virus strain. A standard 0.5 ml injectable dose in most cases contain 15 µg of haemagglutinin (HA) antigen component from each strain, as measured by single radial immunodiffusion (SRD) (J.M. Wood et al.: An improved single radial immunodiffusion technique for the assay of influenza haemagglutinin antigen: adaptation for potency determination of inactivated whole virus and subunit vaccines. J. Biol. Stand. 5 (1977) 237-247; J. M. Wood et al., International collaborative study of single radial diffusion and immunoelectrophoresis techniques for the assay of haemagglutinin antigen of influenza virus. J. Biol. Stand. 9 (1981) 317-330).

In certain circumstances, such as the occurrence of a pandemic influenza strain, it may be desirable to have a vaccine which contains only the single strain. This will help the speed of response to a pandemic situation. The influenza virus strains to be incorporated into influenza vaccine each season are determined by the World Health Organisation in collaboration with national health authorities and vaccine manufacturers.

Currently available influenza vaccines are either inactivated influenza vaccines or live attenuated influenza vaccines. Inactivated influenza vaccines comprise one of three types of antigen preparation: inactivated whole virus, sub-virions where the membrane of the virus particles is disrupted with detergents or other reagents to solubilize the lipid envelope (so-called 'split' vaccine) or subunits vaccines (produced either recombinantly or purified from disrupted viral particles) in particular HA and NA subunit vaccine. Virosomes are another type of inactivated influenza formulation, where the viral membrane is reconstituted following disruption. These inactivated vaccines are generally given parenterally, in particular intramuscularly (i.m.), but some virosome-based formulations and live attenuated vaccines have been administered intranasally.

While the immunogenicity and efficacy of parenterally (e.g. intramuscularly) administered influenza vaccines are generally recognized to be quite high in healthy adults (18-60 years of age) (efficacy estimated around 75%), these parameters are reduced in elderly subjects (>60 years of age) (efficacy estimated around 50%).

As elderly subjects are the people most at risk of severe influenza-induced disease, especially those residing in nursing facilities, this leaves room for significant improvement in influenza vaccine efficacy in the elderly. Thus design of a novel influenza vaccine with increased efficacy for elderly subjects would be an important improvement over the vaccines currently available.

Parenterally administered influenza vaccines are generally immunogenic and well tolerated, however the level of immunogenicity and associated reactogenicity varies between the various compositions with a higher level of immunogenicity and associated reactogenicity generally seen with the more complex formulations (whole virus and split vs subunit and virosome). Thus designing a novel formulation which could substantially improve the immunogenicity of currently commercially available influenza vaccines while maintaining an acceptable reactogenicity profile has remained challenging.

As said above, the currently commercially available influenza vaccines remain the intramuscularly administered split vaccine, whole virus vaccine, subunit injectable or virosomes vaccines. In particular, the split and subunit vaccines are prepared by disrupting the virus particle, generally with an organic solvent or a detergent, and separating or purifying the viral proteins to varying extents. Split vaccines are prepared by fragmentation of whole influenza virus, either infectious or inactivated, with solubilizing concentrations of organic solvents or detergents and subsequent removal of the solubilizing agent and some or most of the viral lipid material. Split vaccines generally contain contaminating matrix (M) protein and nucleoprotein (NP) and sometimes lipid, as well as the membrane envelope proteins (such as HA and NA). Split vaccines will usually contain most or all of the virus structural proteins although not necessarily in the same proportions as they occur in the whole virus. Subunit vaccines on the other hand consist essentially of highly purified viral surface proteins, haemagglutinin (HA) and neuraminidase (NA), which are the surface proteins responsible for eliciting the desired virus neutralising antibodies upon vaccination. Matrix and nucleoproteins are either not detectable or barely detectable in subunit vaccines.

Standards are applied internationally to measure the efficacy of influenza vaccines. The European Union official criteria for an effective vaccine against influenza are set out in the table below. Theoretically, to meet the European Union requirements, an influenza vaccine has to meet only one of the criteria in the table, for all strains of influenza included in the vaccine. However in practice, at least two or more probably all three of the criteria will need to be met for all strains, particularly for a new vaccine such as a new intradermal vaccine. Under some circumstances two criteria may be sufficient. For example, it may be acceptable for two of the three criteria to be met by all strains while the third criterion is met by some but not all strains (e.g. two out of three strains). The requirements (Note for Guidance on harmonisation of requirements for influenza vaccines, CPMP/BWP/214/96, 12 March 1997) are different for adult populations (18-60 years) and elderly populations (>60 years):
a) the following serological assessments should be considered for each strain in adult subjects, aged between 18 and 60, and at least one of the assessments should meet the indicated requirements:
   - number of seroconversions or significant increase in antihaemagglutinin antibody titre > 40%;
   - mean geometric increase > 2.5
   - the proportion of subjects achieving an HI titre ≥40 or SRH titre >25 mm² should be > 70%
b) the following serological assessments should be considered for each strain in adult subjects aged over 60, and at least on e of the assessments should meet the indicated requirements:
   - number of seroconversions or significant increase in antihaemagglutinin antibody titre > 30%;
   - mean geometric increase > 2.0
   - the proportion of subjects achieving an HI titre ≥40 or SRH titre >25 mm² should be > 60%

For a novel influenza formulation to be commercially useful it will not only need to meet those standards, but also in practice it will need to be at least as immunologically efficacious as the currently available vaccines. It will need to be administered using a procedure which is reliable and straightforward for medical staff to carry out. Furthermore, it will also need to be produced by an acceptable process and will of course need to be commercially viable in terms of the amount of antigen and the number of administrations required.

Various approaches for developing such an improved influenza vaccine have been pursued. However, to date none of these approaches have been successfully implemented to produce and commercialize an influenza vaccine with increased efficacy which maintains an acceptable reactogenicity profile, highlighting the significant difficulties in finding such a formulation.

One approach which has been investigated is the use of an influenza virosome vaccine rather than the use of a conventional (i.e. split, sub-unit or whole) vaccine. Virosomes are reconstituted lipid membranes containing antigens from the virus. Virosomes are produced by two major approaches, one approach being based on the addition of exogeneous lipids during the reconstitution of the virosome (Almeida et al., 1975. Lancet 2:899-901; Trudel M. and F. Nadon, 1981. Can J Microbiol. 27:958-62; Ando et al., 1997. J Microencapsul. 14:79-90; Markgraf et al., 2001. Cloning 3:11-21; Gluck, R. and I.C. Metcalfe, 2002. Vaccine 20:B10-16; Mischler, R. and I.C. Metcalfe, 2002. Vaccine 20:B17-23) and the other being based on the formation of virosomes in the absence of addition of exogeneous lipids (Stegmann, T. et al. 1987. EMBO J. 6:2651-9; Huckriede et al., 2003. Vaccine 21:925-31). A virosome vaccine has been shown to be more immunogenic than a commercial trivalent subunit influenza vaccine in geriatric patients (Conne et al. Vaccine, 1999, 15, 1675-1679), although in another study in the elderly the virosome formulation alone was not more immunogenic than a commercially available split vaccine (Ruf et al., 2003. Options for the Control of Influenza V Conference, Okinawa, Japan. Oct 7-11, Immunogenicity and antibody persistence of FLUARIX™ vs. FLUAD^{®} vs. INFLEXAL V^{®} in the elderly).

In another approach towards the development of improved influenza formulations, the addition of adjuvants in various forms to different influenza vaccine compositions and in particular to subunit vaccines, have also been tested (reviewed in O'Hagan, D.T., 1998. J. Pharm Pharmacol. 50:1-10; Gluck R. 1992. Vaccine 10:915-9; Podda A. and G. Del Giudice, 2003. Expert Rev Vaccines 2:197-203; Nerome et al. 1990. Vaccine 8:503-9; Chen, D. et al, 2003. Vaccine 21: 2830-6). However very few of these formulations have advanced in development, presumably due to lack of convincing data for increased immunogenicity and acceptable safety. Although two adjuvanted formulations have been commercialized, they both fail to meet the criteria of better efficacy and acceptable reactogenicity. One adjuvanted formulation developed by Berna was a virosome based formulation to which *E. coli* labile toxin (LT) was added. It was commercialized under the name of Inflexal N®. This formulation was administered by the intranasal route and while immunogenic was found to have an unacceptable safety profile (association with partial facial paralysis) and has been removed from the market. The other adjuvanted formulation which has been commercialized is FLUAD^{®} (Chiron) which is a subunit vaccine adjuvanted with MF59 for parenteral administration. This formulation appears to be relatively well tolerated but has been shown to be no more immunogenic than other commercially available classical vaccines (Ruf et al., 2003, see above).

Saponins are taught in: Lacaille-Dubois, M and Wagner H. (1996. A review of the biological and pharmacological activities of saponins. Phytomedicine vol 2 pp 363-386). Saponins are steroid or triterpene glycosides widely distributed in the plant and marine animal kingdoms. Saponins are noted for forming colloidal solutions in water which foam on shaking, and for precipitating cholesterol. When saponins are near cell membranes they create pore-like structures in the membrane which cause the membrane to burst. Haemolysis of erythrocytes is an example of this phenomenon, which is a property of certain, but not all, saponins.

Saponins are known as adjuvants in vaccines for systemic administration. The adjuvant and haemolytic activity of individual saponins has been extensively studied in the art (Lacaille-Dubois and Wagner, *supra*). For example, Quil A (derived from the bark of the South American tree Quillaja Saponaria Molina), was first described by Dalsgaard et al. in 1974 ("Saponin adjuvants", Archiv. für die gesamte Virusforschung, Vol. 44, Springer Verlag, Berlin, p243-254) to have adjuvant activity. Purified fragments of Quil A have been isolated by HPLC which retain adjuvant activity without the toxicity associated with Quil A (EP 0 362 278), for example QS7 and QS21 (also known as QA7 and QA21). Quil A fractions are described in US 5,057,540 and "Saponins as vaccine adjuvants", Kensil, C. R., Crit Rev Ther Drug Carrier Syst, 1996, 12 (1-2):1-55; and EP 0 362 279 B1. Particulate structures, termed Immune Stimulating Complexes (ISCOMS), comprising Quil A or fractions thereof, have been used in the manufacture of vaccines (Morein, B., EP 0 109 942 B1). These structures have been reported to have adjuvant activity (EP 0 109 942 B1; WO 96/11711). The haemolytic saponins QS21 and QS17 (HPLC purified fractions of Quil A) have been described as potent systemic adjuvants, and the method of their production is disclosed in US Patent No. 5,057,540 and EP 0 362 279 B1. Also described in these references is the use of QS7 (a non-haemolytic fraction of Quil-A) which acts as a potent adjuvant for systemic and mucosal (e.g. intranasal) vaccines. Use of QS21 is further described in Kensil et al. (1991. J. Immunology vol 146, 431-437). Combinations of QS21 and polysorbate or cyclodextrin are also known (WO 99/10008). Particulate adjuvant systems comprising fractions of QuilA, such as QS21 and QS7 are described in WO 96/33739 and WO 96/11711.

Other saponins which have been used in systemic vaccination studies include those derived from other plant species such as Gypsophila and Saponaria (Bomford et al., Vaccine, 10(9):572-577, 1992).

Saponins are also known to have been used in mucosally applied vaccine studies, which have met variable success in the induction of immune responses. Quil-A saponin has previously been shown to have no effect on the induction of an immune response when antigen is administered intranasally (Gizurarson et al. 1994. Vaccine Research 3, 23-29). Whilst, other authors have used this adjuvant with success (Maharaj et al., Can.J.Microbiol, 1986, 32(5):414-20; Chavali and Campbell, Immunobiology, 174(3):347-59). ISCOMs comprising Quil A saponin have been used in intragastric and intranasal vaccine formulations and exhibited adjuvant activity (Mcl Mowat et al., 1991, Immunology, 72, 317-322; Mcl Mowat and Donachie, Immunology Today, 12, 383-385). QS21, the purified fraction of Quil A, has also been described as an oral or intranasal adjuvant (Sumino et al., J. Virol., 1998, 72(6):4931-9; WO 98/56415).

The use of QS21 as an adjuvant is associated with certain disadvantages. For example when QS21 is injected parenterally into a mammal as a free molecule it has been observed that necrosis, that is to say, localised tissue death, occurs at the injection site. Adjuvanted subunit influenza formulations based on saponins have been reported, such as influenza virus haemagglutinin (HA) and neuraminidase (NA) integrated into ISCOMs (Sundquist et al. Vaccine, 1988, 6, 44-48; Barr I.G., and G.F. Mitchell. 1996. Immunol Cell Biol. 74:8-25; Kersten G.F.A., and D.J.A. Crommelin 2003. Vaccine 21:915-920). But despite years of effort, a product acceptable for human use has yet to emerge (Kersten G.F.A., and D.J.A. Crommelin 2003. Vaccine 21:915-920).

Thus finding a formulation which could provide an increased immune response compared to existing licensed influenza vaccines whilst maintaining an acceptable reactogenicity profile remains an area requiring investigation. The present invention sets out to address the need for an improved influenza vaccine.

The present invention relates to the identification of a novel influenza formulation based on the combination of virosomes and saponins which fulfills the criteria of increased immunogenicity and efficacy while maintaining an acceptable reactogenicity profile and be effective in particular in the elderly population.

### STATEMENT OF THE INVENTION

The present inventors have identified a novel composition based on the combination of a virosome preparation and a saponin adjuvant which fulfills the criteria of increased immunogenicity and efficacy while maintaining an acceptable reactogenicity profile.

Accordingly, in a first aspect, the present invention relates to a composition or vaccine comprising an influenza virosome preparation and QS21.

QS21 is an immunologically active saponin fraction having adjuvant activity derived from the bark of the South American tree Quillaja Saponaria Molina, also known as QA21, an HPLC purified fraction from the Quillaja Saponaria Molina tree. This fraction is well known in the art and can be produced according to the method disclosed (as QA21) in US patent No. 5,057,540. Quillaja saponin has also been disclosed as an adjuvant by Scott et al, Int. Archs. Allergy Appl. Immun., 1985, 77, 409.

In another embodiment the composition comprising an influenza virosome and QS21 contains an additional immunostimulant chemical selected from the group consisting of: a Toll like receptors agonist, (in particular Toll like receptor 2 agonist, Toll like receptor 3 agonist, Toll like receptor 4 agonist, Toll like receptor 7 agonist, Toll like receptor 8 agonist and Toll like receptor 9 agonist), aluminium hydroxide, aluminium phosphate, tocopherol, and an oil-in-water emulsion or a combination of two or more of the said adjuvants.

The invention further relates to the use of (1) an influenza virosome preparation and (2) QS21, in the manufacture of a medicament for achieving a protective immune response or reducing the severity of disease caused by influenza virus in a patient, in particular an elderly patient.

Also described is a method for preparing an influenza virosome preparation comprising treating the whole enveloped virus with an ionic, typically an anionic detergent, and, suitably, subsequently adding exogeneous sterol and/or exogeneous lipids during the virosomes reconstitution. In another embodiment, the invention relates to a method for preparing an influenza virosome composition or vaccine comprising combining an influenza virosome preparation, with QS21.

The present invention also relates to an influenza virosome adjuvanted composition or vaccine as defined in the claims, for use in the prophylaxis of a disease caused by influenza virus, in particular influenza infection or disease, in an individual.

The present invention further relates to use of an influenza virosome preparation, and QS21, in the manufacture of a composition or vaccine for the prophylaxis of influenza infection or disease.

Also described is the use of QS21, in the adjuvantation of an influenza virosome preparation.

Also described is a method for the prophylaxis of influenza infection or disease in a subject which method comprises sequential administration or co-administration of an influenza virosome preparation, and of QS21.

A delivery device, in particular an intradermal, intranasal or transcutaneous delivery device, containing an influenza virosome preparation in combination with QS21, is also described.

Other aspects and advantages of the present invention are described further in the following detailed description of the preferred embodiments thereof.

### DESCRIPTION OF FIGURES

Figure 1 - Schematic representation of the process for the preparation of virosomes using Sarcosyl^{®} as detergent
Figure 2 - Yield in total protein and in HA of the splitting process with Sarcosyl^{®}
Figure 3 - Anti-Influenza A/New Caledonia H1N1 Haemagglutination Inhibition Antibody Responses in Mice immunized with various virosome-based formulations.
Figure 4 - Anti-Influenza A/Panama H3N2 Haemagglutination Inhibition Antibody Responses in Mice immunized with various virosome-based formulations.
Figure 5 - Anti-Influenza A/New Caledonia H1N1 Haemagglutination Inhibition Antibody Responses in Mice immunized with various virosome-based formulations.
Figure 6 - Count rate measurements of the fractions collected after sucrose gradient
Figure 7 - HA, Phospholipids and cholesterol recoveries after filtration on 0.2µm
Figure 8 - Protein, HA, Phospholipids and cholesterol yields after dialysis
Figure 9 - Count rate (CR) of the fractions collected after sucrose gradient
Figure 10 - Protein, HA, cholesterol and phospholipid yields after dialysis
Figure 11 - Immunogenicity of a QS21-adjuvanted and un-adjuvanted virosome vaccine

### DETAILED DESCRIPTION

In all embodiments of the present invention, the enveloped virus may is Influenza virus.

According to the present invention, the saponin adjuvant is the derivative of *saponaria molina* quil A, known as QS-21. An advantage of such an adjuvanted virosome vaccine as compared to conventional (e.g. sub-unit) vaccine adjuvanted with a saponin is the capacity of the endogeneous (i.e. from the virus) sterol to quench the lytic activity of the saponin and thereby quench the local reactogenicity associated with the saponin adjuvant. Typically an influenza virus contains about 20 to 24% lipids, amongst which about 10-13% phospholipids, about 6 to 8% sterol and about 1 to 2% glycolipids (Kilbourne E.D., The influenza viruses and influenza, Academic press, New York, San Francisco, London, 1975). The adjuvanted influenza virosome vaccine according to the invention will furthermore show an increased immunogenicity as compared to the commercially available split vaccine, un-adjuvanted.

The ratio of QS21 (optionally in its detoxified liposomal stucture as described in WO 96/33739):virosome (as measured by the HA content) will typically be in the order of 100:1 to 1:100 (w/w). Suitable ranges are from 50:1 to 1:50 (w/w), 10:1 to 1:10 (w/w), such as 5:1, 4:1, 3.33:1, 3:1, 2:1 (w/w), 1:1 (w/w) which is preferred.

In a specific embodiment, QS21 is provided in its less reactogenic composition where it is quenched with an exogenous sterol, such as cholesterol for example. Several particular forms of less reactogenic compositions wherein QS21 is quenched with an exogenous cholesterol exist. In one embodiment, the saponin/sterol is in the form of an ISCOM, comprising suitably a non toxic fraction of Quil A. These structures have been reported to have adjuvant activity (WO 96/11711). In another embodiment, alternative particulate structures containing a saponin and a sterol, other than ISCOMs which are also less toxic than the saponin alone, are contemplated which are known to form a liposome structure (WO 96/33739).

The liposomes suitably contain a neutral lipid, for example phosphatidylcholine, which is suitably non-crystalline at room temperature, for example eggyolk phosphatidylcholine, dioleoyl phosphatidylcholine or dilauryl phosphatidylcholine. The liposomes may also contain a charged lipid which increases the stability of the lipsome-QS21 structure for liposomes composed of saturated lipids. In these cases the amount of charged lipid is suitably 1-20% w/w, preferably 5-10%. The ratio of sterol to phospholipid is 1-50% (mol/mol), suitably 20-25%.

Liposomal structures or alternatively ISCOM 'cage-like' structures exist. A particular form of such a composition is as described in WO 96/33739. Another suitable form of such a composition is an adjuvant formulation comprising a saponin and a sterol, characterised in that the adjuvant is in the form of an ISCOM. Suitably the ISCOM is free of additional detergent, other than the saponin as described in WO 00/07621.

ISCOMs are conventionally formed through two steps (e.g. as described in EP 0 109 942): 1, solubilisation of membrane and membrane proteins with detergent; 2, removal of solubilising agent by several means whilst at the same time contacting the membrane components with the saponin whose concentration is at least equal to the critical micellular concentration of the saponin, or removing the solubilising agent and directly transferring the antigen to the solution of saponin. US Patent No. 4,578,269 teaches particular methods of separating the antigen from the solubilising agent. These methods include, amongst others: centrifugation through a gradient of solubilisation agent into an inverse gradient of saponin; or alternatively the solubilised antigen can be mixed with saponin followed by centrifugation of the mixture and dialysis to remove excess detergent.

EP 0 242 380 teaches of an improvement in this manufacturing process. This patent tells how the addition of lipids to the process prevents the formation of antigen/glycoside micelles, and ensures that the antigen/glycoside structures are all ISCOM-like. The specification states that the lipids may be added at any stage, at a molar ratio of at least 0.1:1 of lipids to antigen, and preferably 1:1. Examples of lipids include cholesterol and phosphatidyl choline.

Alternatively said ISCOM structure is made by the process disclosed in WO 00/07621 (which is incorporated by reference) which is characterised by the fact that it is free of additional detergents, other than the saponin.

Suitable sterols include β-sitosterol, stigmasterol, ergosterol, ergocalciferol and cholesterol, which is preferred. These sterols are well known in the art, for example cholesterol is disclosed in the Merck Index, 11th Edn., page 341, as a naturally occurring sterol found in animal fat. Said composition has the advantage of maintaining the adjuvant effect of the virosome associated saponins whilst showing a reduced reactogenicity as compared to non virosomal influenza formulations containing a saponin adjuvant.

Compositions of the invention comprising QS21 and exogenous cholesterol show a further decreased reactogenicity when compared to compositions in which the exogenous cholesterol is absent, while the adjuvant effect is maintained. Reactogenicity studies may be assessed according to the methods disclosed in WO 96/33739.

By exogenous sterol is meant a sterol which is not endogenous to the virus but is added to the virosome preparation or subsequently at the moment of formulation. Typically, the exogenous sterol may be supplemented to the virosome preparation during the method of production of the virosome, or alternatively, may be added during subsequent formulation of the virosome preparation with the saponin adjuvant, by using, for example, the saponin in its form quenched with the sterol. Suitably the exogenous sterol is associated to the saponin adjuvant as described in WO 96/33739.

The ratio of QS21 : exogenous sterol will typically be in the order of 1:100 to 1:1 (w/w), suitably between 1:10 to 1:1 (w/w), and preferably 1:5 to 1:1 (w/w). Suitably excess sterol is present, the ratio of QS21:sterol being at least 1:2 (w/w). The sterol is suitably cholesterol. Typically for human administration QS21 and sterol will be present in a vaccine in the range of about 1 µg to about 100 µg, suitably about 10 µg to about 50 µg per dose.

Other useful saponins disclosed are derived from the plants *Aesculus hippocastanum* or *Gyophilla struthium.* Other saponins which have been described in the literature include Escin, which has been described in the Merck index (12^{th} ed: entry 3737) as a mixture of saponins occuring in the seed of the horse chestnut tree, Lat: *Aesculus hippocastanum.* Its isolation is described by chromatography and purification (Fiedler, Arzneimittel-Forsch. 4, 213 (1953)), and by ion-exchange resins (Erbring et al., US 3,238,190). Fractions of escin have been purified and shown to be biologically active (Yoshikawa M, et al. (Chem Pharm Bull (Tokyo) 1996 Aug;44(8):1454-1464)). Sapoalbin from *Gypsophilla struthium* (R. Vochten et al., 1968, J. Pharm.Belg., 42, 213-226) has also been described in relation to ISCOM production for example.

The virosome preparation suitably contains an exogenous neutral lipid, for example phosphatidylcholine, which is preferably non-crystalline at room temperature, for example eggyolk phosphatidylcholine, dioleoyl phosphatidylcholine or dilauryl phosphatidylcholine. Dioleoyl phosphatidylcholine is preferred. The ratio of sterol : phospholipid is 1-50% (mol/mol), suitably 20-25%.

By exogenous phospholipid is meant a phospholipid which is not endogenous to the virus but is added to the preparation subsequently. The exogenous phospholipid may be supplemented to the virosome preparation during the method of production of the virosome, ot alternatively, may be added during subsequent formulation of the virosome preparation with the saponin/cholesterol adjuvant.

Within other embodiments of the invention, the immunogenic composition additionally comprises another adjuvant, suitably one that induces an immune response predominantly of the Th1 type. Suitable TH-1 inducing adjuvants are selected from the group of adjuvants comprising: a Toll like receptors agonist, (in particular Toll like receptor 2 agonist, Toll like receptor 3 agonist, Toll like receptor 4 agonist, Toll like receptor 7 agonist, Toll like receptor 8 agonist and Toll like receptor 9 agonist), aluminium hydroxide, aluminium phosphate, tocopherol, and an oil-in-water emulsion or a combination of two or more of the said adjuvants.

Suitably the additional adjuvant is a Toll like receptors agonist. The additional adjuvant may be a Toll like receptor 9 agonist, or the adjuvant may be a Toll like receptor (TLR) 4 agonist such as a lipid A derivative particularly monophosphoryl lipid A or more particularly 3 Deacylated monophoshoryl lipid A (3D-MPL).

MPL^{®} adjuvants are available from Corixa Corporation (Seattle, WA; see, for example, US Patent Nos. 4,436,727; 4,877,611; 4,866,034 and 4,912,094) and primarily promote CD4+ T cell responses with an IFN-g (Th1) phenotype. MPL^{®} can be produced according to the methods disclosed in GB 2 220 211 A. Chemically it is a mixture of 3-deacylated monophosphoryl lipid A with 3, 4, 5 or 6 acylated chains. Preferably in the compositions of the present invention small particle 3D-MPL is used. Small particle 3D-MPL has a particle size such that it may be sterile-filtered through a 0.22µm filter. Such preparations are described in WO 94/21292.

Suitable compositions of the invention are those wherein liposomes are initially prepared without MPL (as described in WO 96/33739), and MPL is then added, suitably as 100 nm particles. The MPL is therefore not contained within the vesicle membrane (known as MPL out). Compositions where the MPL is contained within the vesicle membrane (known as MPL in) are also described. The antigen can be contained within the vesicle membrane or contained outside the vesicle membrane. Suitably soluble antigens are outside and hydrophobic or lipidated antigens are either contained inside or outside the membrane.

Synthetic derivatives of lipid A are known and thought to be TLR 4 agonist including, but not limited to:
**OM174** (2-deoxy-6-o-[2-deoxy-2-[(R)-3-dodecanoyloxytetra-decanoylamino]-4-o-phosphono-β-D-glucopyranosyl]-2-[(R)-3-hydroxytetradecanoylamino]-α-D-glucopyranosyldihydrogenphosphate) (WO 95/14026);
**OM294** DP (3S, 9 R) -3--[(R)-dodecanoyloxytetradecanoylamino]-4-oxo-5-aza-9(R)-[(R)-3-hydroxytetradecanoylamino]decan-1,10-diol,1,10-bis(dihydrogenophosphate) (WO 99/64301 and WO 00/0462)
**OM197** MP-Ac DP ( 3S-, 9R) -3-[(R) -dodecanoyloxytetradecanoylamino]-4-oxo-5-aza-9-[(R)-3-hydroxytetradecanoylamino]decan-1,10-diol,1 -dihydrogenophosphate 10-(6-aminohexanoate) (WO 01/46127)

Immunostimulatory oligonucleotides or any other Toll-like receptor (TLR) 9 agonist may also be used. Immunostimulatory DNA sequences are described, for example, by Sato et al., Science 273:352, 1996. Suitable oligonucleotides for use in adjuvants or vaccines of the present invention are CpG containing oligonucleotides, preferably contain two or more dinucleotide CpG motifs separated by at least three, more preferably at least six or more nucleotides. The oligonucleotides are typically deoxynucleotides. The internucleotide in the oligonucleotide may be phosphorodithioate, or suitably a phosphorothioate bond, although phospho-diester and other internucleotide bonds are envisaged including oligonucleotides with mixed internucleotide linkages. The CpG oligonucleotides that may be utilised in the present invention may be synthesized by any method known in the art (eg EP 468520). Conveniently, such oligonucleotides may be synthesized utilising an automated synthesizer. Methods for producing phosphorothioate oligonucleotides or phosphoro-dithioate are described in US5,666,153, US5,278,302 and WO 95/26204.

Examples of suitable oligonucleotides have the following sequences. The sequences suitably contain phosphorothioate modified internucleotide linkages.
OLIGO 1(SEQ ID NO:1): TCC ATG ACG TTC CTG ACG TT (CpG 1826)
OLIGO 2 (SEQ ID NO:2): TCT CCC AGC GTG CGC CAT (CpG 1758)
OLIGO 3(SEQ ID NO:3): ACC GAT GAC GTC GCC GGT GAC GGC ACC ACG
OLIGO 4 (SEQ ID NO:4): TCG TCG TTT TGT CGT TTT GTC GTT (CpG 2006 also known as CpG 7909)
OLIGO 5 (SEQ ID NO:5): TCC ATG ACG TTC CTG ATG CT (CpG 1668)

Alternative CpG oligonucleotides may comprise the preferred sequences above in that they have inconsequential deletions or additions thereto.

Examples of a TLR 2 agonist include peptidoglycan or lipoprotein. Imidazoquinolines, such as Imiquimod and Resiquimod are known TLR7 agonist. Single stranded RNA is a known TLR8 agonist, whereas double stranded RNA and poly IC are exemplary of TLR 3 agonists.

Particularly suitable adjuvants are combinations of 3D-MPL and QS21 (WO 94/00153), oil in water emulsions comprising 3D-MPL and QS21 (WO 95/17210, WO 98/56414), or 3D-MPL formulated with other carriers (EP 0 689 454 B1). Another particularly suitable enhanced formulation involves the combination of a CpG-containing oligonucleotide with QS21, particularly the combination of CpG and QS21 as disclosed in WO 00/09159 and in WO 00/62800. Other suitable adjuvant systems comprise a combination of 3D-MPL, QS21 and a CpG oligonucleotide as described in US6558670, US6544518. QS21 is preferably provided in its less reactogenic composition where it is quenched with cholesterol, as described in WO 96/33739.

Accordingly, in another embodiment, the formulation according to the invention comprises (1) an influenza virosome preparation and (2) a combination of QS21, more preferably in its quenched form with cholesterol, with one or more of the following adjuvants selected from the list consisting of: a Toll like receptors agonist (in particular Toll like receptor 2 agonist, Toll like receptor 3 agonist, Toll like receptor 4 agonist, Toll like receptor 7 agonist, Toll like receptor 8 agonist and Toll like receptor 9 agonist), aluminium hydroxide, aluminium phosphate, tocopherol, and an oil-in-water emulsion or a combination of two or more of the said adjuvants.

Alternatively QS21 within the composition according to the invention may be combined with vaccine vehicles composed of chitosan or other polycationic polymers, polylactide and polylactide-co-glycolide particles, poly-N-acetyl glucosamine-based polymer matrix, particles composed of polysaccharides or chemically modified polysaccharides, liposomes and lipid-based particles, particles composed of glycerol monoesters, etc. QS21 may also be formulated in the presence of cholesterol to form particulate structures such as liposomes or ISCOMs. Furthermore, QS21 may be formulated together with a polyoxyethylene ether or ester, in either a non-particulate solution or suspension, or in a particulate structure such as a paucilamelar liposome or ISCOM. QS21 may also be formulated with excipients such as Carbopol^{R} to increase viscosity, or may be formulated in a dry powder form with a powder excipient such as lactose.

The influenza antigen can be grown in any suitable substrate such as eggs or cells (such as MDCK cells, for example). Virosomes can be prepared as described by R. Gluck, Vaccine, 1992, 10, 915-920, or Stegmann et al 1987, EMBO Journal 6, 2651-2659. Influenza virosomes may be prepared from a fraction of the detergent-treated virus containing HA and/or NA envelope proteins (i.e. de-capped virus, following the treatment with a decapping agent such as octylglucopyranosiee, OGP, and subsequent recovery of the supernatant containing at least the major envelope protein HA). Alternatively influenza virosomes can be prepared from a splitted virus containing additional proteins to HA and NA. This starting material is preferred as this presents the advantage that the antigen complexity is closer to that of the entire virus. Suitable splitting or de-capping agents are ionic, non-anionic and amphiphilic detergents. Typically non-ionic detergents such as C12E8, C12E9, octylglucopyranosiee (OGP) or Plantacare^{®} are suitable for preparation of virosomes according to the invention. Advantageously the virosomes can be prepared using ionic, preferably anionic detergents, for example following the methodology detailed in Example 1. In particular, Sarcosyl^{®} (lauryl sarcosinate or sodium N-dodecanoyl-N-methylglycinate) is a suitable anionic detergent. Optionally, an additional step is included after the solubilisation step, which consists in the addition of exogeneous sterol during the virosome reconstitution. This method is preferred. Typically sterols for inclusion in the method include β-sitosterol, stigmasterol, ergosterol, ergocalciferol and cholesterol, which is particularly suitable. Alternatively, an additional step is included after the solubilisation step, which consists in the addition of exogeneous lipids during the virosome reconstitution. Typical phospholipids are phosphatidylcholine or derivative thereof, for example eggyolk phosphatidylcholine, dioleoyl phosphatidylcholine (DOPC) or dilauryl phosphatidylcholine. Suitably a mixture of both phospholipids and sterol is used. The preferred sterol is cholesterol, and the preferred phospholipid is dioleyl phosphatidylcholin (DOPC). The method as described will further include a step to remove the detergent, thereby leading to the formation of the virosomes. Typically the detergent removal step is dialysis. Suitably ultrafiltration is performed as this method is compatible with large scale production. This method leads to satisfactory protein yields being produced whilst having the advantage that all the parameters are fixed and controlled.

Optionally the virosome preparation, in particular but not exclusively the HA component of the B strain influenza of the preparation, may require further stabilisation. Suitable stabilisers are α-tocopherol or derivatives thereof such as α-tocopherol succinate. Other suitable tocopherol derivatives for use in the invention include D-α tocopherol, D-δ tocopherol, D-γ tocopherol and DL-α-tocopherol. Suitable derivatives of tocopherols that may be used include acetates, succinates, phosphoric acid esters, formiates, propionates, butyrates, sulfates and gluconates. Alpha-tocopherol succinate is particularly suitable. The α-tocopherol or derivative is present in an amount sufficient to stabilise the haemagglutinin component of the influenza preparation.

Accordingly there is also described a process for preparing influenza virosomes comprising treating the whole enveloped virus with an ionic, suitably an anionic detergent, typically but not exclusively Sarcosyl^{®} detergent, separating the pellet from the supernatant containing the split preparation, and subsequently admixing the split preparation with a mixture of exogeneous phospholipids and sterol, preferably cholesterol. The detergents are used at a ratio detergent:protein (as measured by the HA content) of 100 : 1 to 1 : 1 (w/w), suitably at a ratio of 50 : 1 to 1 : 1 (w/w), such as a ratio of 20 : 1 to 1 : 1, in particular at a ratio of 10 : 1 to 1 : 1 (w/w). A ratio of about 10 : 1 (w/w) is particularly preferred. Other suitable detergents for the preparation of virosomes are non-ionic detergents such as C12E8, C12E9, octylglucopyranosiee (OGP) or Plantacare^{®}.

Alternatively, other sterols may be used in admixture with the phospholipids, such as sitosterol (e.g. β-Sitosterol), stigmasterol, ergosterol, and ergocalciferol. Typically the mixture of phospholipids and sterol is used at a ratio of phospholipids : sterol (preferably cholesterol) of 1:10 to 10:1 (w/w), suitably at a ratio of 4:1 (w/w), such as a ratio of 1:1. Typically the detergent :total protein (as measured by the BCA Compat-Able™ test, ref: 23215 from Pierce) ratio is from 1000:1 1 to 1:1 (w/w), preferably 100 : 1 (w/w), more preferably 20 : 1. Suitably this ratio is 10 (w/w).

The influenza vaccine for use according to the invention is suitably a multivalent influenza vaccine comprising two or more strains of influenza. Typically it is a trivalent vaccine comprising three strains. Conventional influenza vaccines comprise three strains of influenza, two A strains and one B strain. A standard vaccine dose usually contains from 3 µg to 45 µg of HA component as measured by the SRD assay. A standard 0.5 ml injectable dose in most cases contains 15 µg of haemagglutinin antigen component from each strain, as measured by single radial immunodiffusion (SRD). However, monovalent vaccines, such as vaccines based on a strain associated with a pandemic outbreak, or which have the potential of being associated with a pandemic situation, are not excluded from use according to invention. A monovalent, pandemic influenza vaccine will most likely contain influenza antigen from a single A strain.

The influenza composition or vaccine for use according to the invention suitably meets some or all of the EU criteria for influenza vaccines as set out hereinabove, such that the vaccine is approvable in Europe. Preferably, at least two out of the three EU criteria are met, for all strains of influenza represented in the vaccine. More preferably, at least two criteria are met for all strains and the third criterion is met by all strains or at least by all but one of the strains. Most preferably, all strains present meet all three of the criteria.

The compositions of the present invention can be administered via any appropriate delivery route, including parenteral, and mucosal routes. This composition is particularly, though not exclusively, suitable for parenteral administration, typically for intramuscular, sub-cutanesous or intradermal injection. This vaccine is also suitable for mucosal administration including intranasal administration.

For the avoidance of doubt the terms 'comprising', 'comprise' and 'comprises' herein is intended by the inventors to be optionally substitutable with the terms 'consisting of', 'consist of', and 'consists of', respectively, in every instance.

The invention will be further described by reference to the following, non-limiting, examples:

### Example I - Preparation of QS21 adjuvanted virosomes

Whole influenza virus is disrupted by a detergent that can be, for the most part, subsequently removed. Insoluble material is discarded by centrifugation. Soluble material containing at least HA is added to a mixture of phospholipid (PL) and cholesterol. Virosomes are then formed following detergent elimination. If needed, non integrated protein can be discarded after purification by sucrose gradient. Purified material or non purified material can then be adjuvanted by the addition of QS21. Exogeneous PL and cholesterol are added during the process to obtain virosomes that decrease or abrogate the lytic activity associated to the saponin adjuvant. This effect on the reactogenicity of the saponin adjuvant is achieved due to the presence of cholesterol. When QS21 saponin is added to the virosomes preparation, to obtain a specific cholesterol to QS21 ratio, the local reactogenicity of QS21 is partially or totally inhibited.

### I.1. Preparation of virosomes using Sarcosyl^{®} as splitting agent

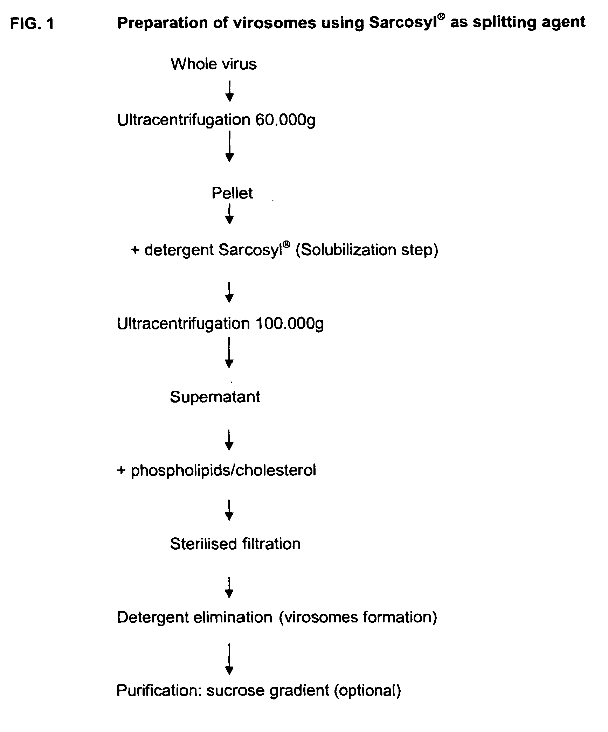

A schematic representation of the preparation of virosomes is illustrated in Figure 1.

Virosomes composed of reconstituted viral envelope, exogeneous lipids (phospholipids and cholesterol) and influenza protein(s) (such as HA and NA) are prepared according to the following method.

Sarcosyl^{®} is used as a splitting agent, i.e. HA and the other influenza proteins are recovered in the supernatant after centrifugation of the treated virus.

BPL (Beta propiolactone) - inactivated whole virus is pelleted following 1H centrifugation at 60.000g and is subsequently disrupted following the action of a detergent. This can also be performed in batch mode. A detergent: protein ratio of 10 (w/w) is used. In this example Sarcosyl^{®} detergent (Sigma L-9150) is used. After 2 hours at room temperature, the suspension is centrifuged for 1 hour at 100,000 g and the insoluble material is discarded. The contact time with the detergent may range from 1 hour to 24 hours.

In parallel, a mixture of phospholipids and cholesterol in chloroform or ethanol (containing or not Sarcosyl^{®}) at a ratio of 4 (w/w) is dried on a rotary evaporator. The resulting film is reconstituted with the solubilised material and shaken for at least 2H at RT. Phospholipid/Cholesterol/HA targeted composition are for example 1000/250/45 or 500/125/45 (w/w/w).

The resulting preparation is then filtered on 0.2µm and is transferred in a slide-A-Lyzer cassette (10000MWCO) to dialyze against the desired buffer. In this case PBS pH 7.4 was used. Several buffer changes are done. During this dialysis step particles are formed to which HA is associated.

Typically, particles (virosomes) of about 100 nm are obtained with this process as measured by a Malvern Zetasizer 3000HS (dynamic light scattering) under the following conditions: laser wavelength: 532 nm; laser power: 50 mW; scattered light detected at 90°; T°: 25°C; z average diameter: by cumulants analysis.

5 to 45% Sucrose gradient is performed to analyze the virosome sample. The sample is applied on the gradient and is centrifuged at 34000 rpm for 16H (SW41 Ti rotor). Fractions of 1 ml are then collected.

SDS-Page gel is run and is silver stained. Pattern indicates that HA is present in the first fractions of the gel (corresponding to the lowest sucrose density), where phospholipids and cholesterol are also found. Those fractions contain the virosomes. HA is also detected in some fractions with higher density. This corresponds to unbound HA.

A purification step can be added to further remove the non associated proteins. A sucrose gradient (under the same conditions as described above) is run. Fractions at the top of the gradient are kept and pooled. Fractions corresponding to unbound material are discarded.

This method leads to a high yield of HA associated to the virosome structure in the range of 30 to 80% (preferably in the range of 70 to 80%) as measured after detergent elimination (e.g. by dialysis or ultrafiltration) step. Protein yields are conveniently measured by the BCA^{™} method (Pierce kit 23225) preceded or not by a treatment with Compat-Able™ (ref: 23215 from Pierce) or alternatively using the Lowry method. HA concentration is measured by single radial immunodiffusion (SRD) (method recommended by WHO) (J.M. Wood et al.: An improved single radial immunodiffusion technique for the assay of influenza haemagglutinin antigen: adaptation for potency determination of inactivated whole virus and subunit vaccines. J. Biol. Stand. 5 (1977) 237-247; J. M. Wood et al., International collaborative study of single radial diffusion and immunoelectrophoresis techniques for the assay of haemagglutinin antigen of influenza virus. J. Biol. Stand. 9 (1981) 317-330).

### I.2. Comparative experiment using alternative detergents

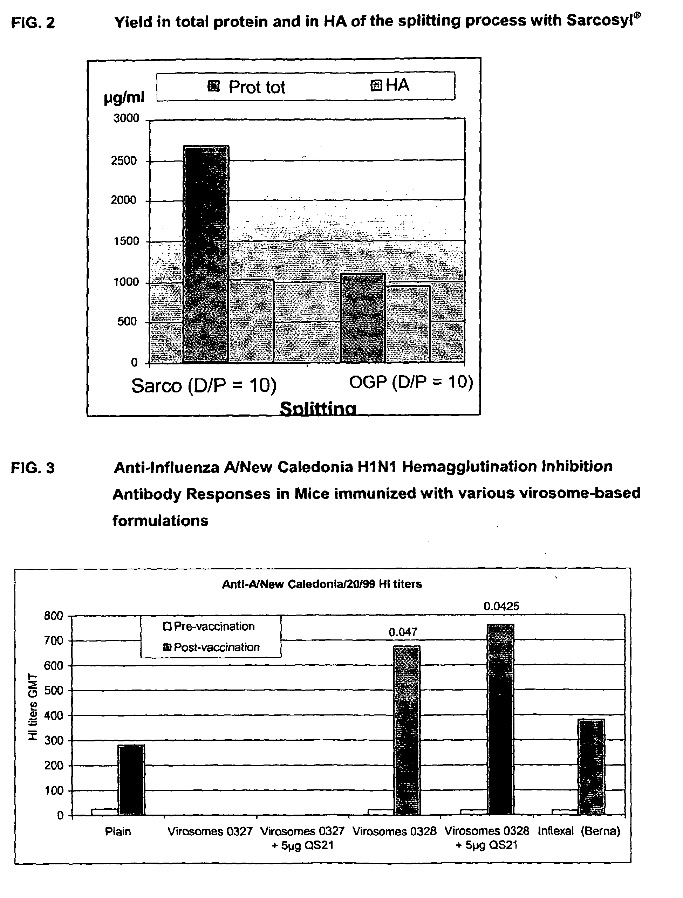

Comparative experiments have been done with octylglucopyranoside (OGP) or Triton X-100 (Octylphenol-polyethylene glycol ether, a non-ionic detergent), or C12E8 (octaethyleneglycol mono n-dodecyl ether, a nonionic detergent, Sigma P-8925) or C12E9 (polyoxyethylene-9-lauryl ether, a nonionic detergent, Sigma P-9641), or Plantacare® (Plantacare® 2000 UP, a C8-C16 alkyl polyglycoside, a non-ionic detergent available from Henkel KGaA) in lieu of Sarcosyl^{®} as detergent. OGP is a decapping agent, i.e. proteins anchored in the membranes (HA and NA) are present after centrifugation of the treated virus. Table 1 and Figure 2 below illustrate the protein content (yield) as measured by the BCA method (Pierce kit 23225 preceded by a treatment with Compat-Able™ ref: 23215 from Pierce), performed on the whole virus before (Table 1, first line, 100%) and after splitting. The measures performed after splitting are performed on the supernatant after the centrifugation step, see lines 2 to 8 in Table 1). HA yields have been measured using the SRD test.

This assay was done in "batch" mode (i.e. on the whole virus not pelleted before contact with detergent) with the B/Shangdong strain BPL (Beta propriolactone) inactivated.

**Table 1**

| Sample | | Protein Yield % | HA yield % |
|---|---|---|---|
| Whole B/Shangdong | | 100 | 100 |
| Sarco | D/P 10 | 90 | 70 |
| C12E8 | D/P 10 | 64 | 84 |
| | D/P 2.5 | 75 | 93 |
| Triton X-100 | D/P 10 | 66 | 88 |
| | D/P 2.5 | 64 | 87 |
| OGP | D/P 10 | 61 | 79 |
| | D/P 2.5 | 27 | 23 |

| | | | |
|---|---|---|---|
| D/P: detergent: protein ratio (w/w) | | | |

### I.3. Adjuvantation of virosomes with QS21

Virosomes are mixed with QS21 to obtain a cholesterol : QS21 ratio of 10 : 1 to 1 : 1 (w/w). QS21 lytic activity is measured with red blood cells to verify that it is inhibited.

Typically, the vaccine formulation contains 3 x 15 µg of HA in virosomes (made from H1N1, H3N2 and B Strains) adjuvanted with 50 µg of QS21 in 500 µl. The final cholesterol : QS21 ratio is 10 : 1 to 1 : 1 (w/w).

### I.4. Assay of the haemolytic activity of the adjuvanted virosomes

Formulations containing virosomes adjuvanted with QS21 are tested for their lytic activity. These results are shown in Table 2A and 2B.

### I.4.1. A. Virosomes prepared according to Example I.1.

Table 2A shows data obtained with virosome preparations made according to example I.1. These preparation have seen exogeneous phospholipids and sterol during theit production process. The formulations contain 45µg HA and 50µg QS21 per 500µl dose. The phospholipid and cholesterol contents vary from one formulation to the other according to Table 2A.

The lytic activity is tested against red blood cells and lysis, when it occurs, is measured by OD at 540nm, according to the procedure detailed below:
Red blood cells are washed 3 times by low speed centrifugation of the blood followed by resuspension of the red cells in PBS to the original volume. After washing, the red cells pellet is diluted 10 times in PBS.
QS21 references are prepared: 1, 2.5 and 5 µg of QS21 are mixed with PBS to reach a final volume of 900 µl. For the samples to be tested, the equivalent of 2.5 and 5 µg of QS21 is taken and PBS is added to reach a volume of 900 µl.
100 µl of red blood cells (diluted 10 times) are added to each reference and sample. The tubes are shaken very gently and let for 30 minutes at room temperature (RT). The tubes are then centrifuged at 2000 rpm (CS-6R centrifuge- Beckman) for 5 minutes.
The OD of the supernatant is read at 540nm.

Results obtained with virosomes produced according to Example I.1 made in the presence of exogeneous cholesterol are illustrated in Table 2A. The results show that such virosomes are able to quench QS21 lytic activity and their performance is correlated to the cholesterol : QS21 ratio.

**Table 2A - virosomes prepared according to Example I.1**

| Formulation | | | | OD |
|---|---|---|---|---|
| DOPC µg | Cholesterol µg | HA µg | Cholesterol : QS21 | |
| 1000 | 250 | 45 | 5 | 0.085 |
| 250 | 62.5 | 45 | 1.24 | 0.148 |
| 100 | 25 | 45 | 0.5 | 0.34 |
| 100 | 0 | 45 | NA | 1.81 |

| | | | | |
|---|---|---|---|---|
| NA = not applicable as cholesterol is absent | | | | |

### I.4.2. Commercially available virosomes Inflexal V® (Berna).

500µl Inflexal V® vaccine, has been adjuvanted by the addition of 50 µg QS21. The resulting composition of this mixture is: +/-117µg of Lecithin, 45µg HA, 50µg QS21 in 525µl. The lytic activity of QS21 in this formulation has been compared to the lytic activity of QS21 alone.

Practically, a volume of the formulation "Inflexal V + QS21" containing the equivalent of 2.5µg of QS21 is added to PBS pH 7.4 to reach a final volume of 900 µl. In parallel, 2.5µg QS21 is taken and also diluted to 900µl with PBS.
100µl of red blood cells (10x diluted) are added to those 2 samples as described above. Samples are let for 30 minutes at RT. After centrifugation at 2000 rpm, the OD of the supernatant is red at 540nm.

**Table 2B**

| QS21 | OD |
|---|---|
| 2.5µg | 1.63 |
| 2.5µg in the Inflexal V® virosome formulation described above | 1.09 |

Table 2B indicates that the lytic activity of QS21 is decreased by the addition of Inflexal V® virosomes, showing that the endogeneous sterol present in the virus is able to decrease the lytic activity of the QS21 adjuvant.

### Example II - Immunogenicity experiments with QS21-adjuvanted virosomes

Immunogenicity of the virosome formulations was assessed in a primed mouse model aimed to reproduce more closely the situation observed in elderly humans, where they have previously encountered influenza antigens, but do not have a protective response pre-vaccination. Two experiments have been carried out.

### II.1. Plan of experiment #1

Mice were primed at day 0 with 5 µg of whole trivalent formalin-inactived influenza (A/New Caledonia/20/99 H1N1, A/Panama/2007/99 H3N2, B/Johannesburg/5/99) administered by the intranasal route.

At day 63, mice were vaccinated IM with:
- Group A: (trivalent split vaccine control): 1.5 µg per strain (HA) of split trivalent vaccine (see above composition) - so-called 'Plain'
- Group B: monovalent virosomes with 1.5 µg (HA) (A/Panama/2007/99, H3N2) - so called 'Virosomes 0327'
- Group C: monovalent virosomes with 1.5 µg (HA) (A/Panama/2007/99, H3N2) + 5 µg QS-21 - so called 'Virosomes 0327 + QS21'
- Group D: monovalent virosomes with 1.5 µg (HA) of split trivalent vaccine (A/New Caledonia/20/99, H1N1) - so called 'Virosomes 0328'
- Group E: monovalent virosomes with 1.5 µg (HA) of split trivalent vaccine (A/New Caledonia/20/99, H1N1) + 5 µg QS-21 - so called 'Virosomes 0328 + QS21'
- Group F: Inflexal V^{®} (Berna, trivalent): 1.5 µg per strain (HA) of virosome trivalent vaccine (A/New Caledonia/20/99 H1N1, A/Moscou/10/99 H3N2, B/Hong Kong/330/2001).
These virosomes were obtained commercially (Inflexal V, Berna Biotech, Berne Switzerland).

Virosomes injected in mice of groups B-E were prepared according to the following methods (see II.2):

### II.2. Preparation of the virosomes

### II.2.1. Virosomes 0328:

11 ml of BPL-inactivated whole A/New Caledonia/20/99, containing 704 µg HA/ml are centrifuged for 1H at 60.000g at 20°C. Supernatant is discarded. Sarcosyl 100 mM in PBS pH 7.4 is added to the pellet to reach a final detergent : protein ratio (w:w) of 10. The pellet is triturated to detach it from the vial wall. The mixture is than shaken (orbital shaking) for 2H at room temperature. Centrifugation at 100 000 g is done for 1H at 20°C. The supernatant (so-called split preparation) is kept.

In parallel, DOPC (60 mg) and Cholesterol (15 mg) solubilized in chloroform are mixed in a Corex tube and chloroform is evaporated (with a Büchi rotary evaporator). The dried lipid film is reconstituted with 3 ml of the split preparation. PBS pH7.4 (1.5ml) is also added to obtain a greater volume. The Corex tube is shaken (orbital shaking) for 3H at RT to allow mixed micelles formation.

The mixture is then filtered on 0.2µm (Millex- GV; 0.22µm Filter unit; ref: SLGV013SL). Dialysis in SPECTRA/POR® membrane (12-14000MWCO; Φ6.4mm, ref 132676) is performed against PBS pH 7.4 for 3 days with 3 buffer changes. After dialysis, a size of 84 nm (as measured with the Zetasizer 3000HS) is obtained with a polydispersity of 0.09.

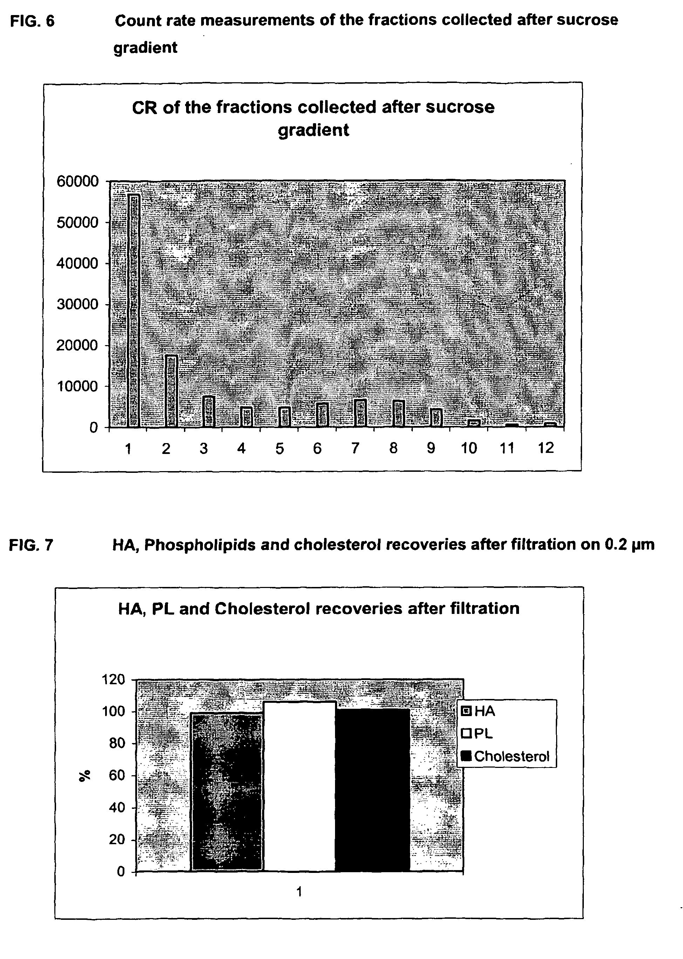

A purification step is done on 5-45% sucrose gradient: 10 tubes are prepared (in 14x89 mm Ultra Clear tubes) on which 400 µl of the sample are layered. Gradients are run for 17H (SW41Ti rotor, 34000rpm, 10°C). Fractions of 1 ml are then collected by the top (fraction 1 = F1 is the top of the gradient, F12 is the bottom of the gradient). The Count Rate (CR) is measured (see figure 6) and indicates where particles are present. Most particles are present in fraction 1 (F1 ). An SDS-Page gel is also performed and this one indicates that HA is also present in this fraction. Fraction 1 of each gradient are pooled together to obtain a large quantity of F1. Fractions 2 to 4 of each gradient are pooled together (Pool F2,3,4), as well as fractions 7 to 9 (Pool F7,8,9). The pools are dialysed against PBS to eliminate residual sucrose. Phospholipid (PL), Cholesterol and HA content are measured. Phospholipid content is measured by the MPR2 kit (Roche, Ref: 691844), Cholesterol by a Boehringer Mannheim kit (ref: 0139050) and HA by SRD. The results indicate that F1 contains PL, Cholesterol and HA as well as pool F2,3,4 meaning that virosomes are present in those fractions. Pool F7,8,9 contains HA but does not contain PL nor cholesterol. This pool corresponds to unassociated proteins.

The final composition of fraction F1 from exp 0328 subsequently injected into the animals is: 6160 µg PU 1805 µg Chol/ 45µg HA.

### II.2.2. Preparation of Virosomes 0327:

They are prepared in the same way as 0328 but with the BPL-inactivated whole A/Panama/2007/99 virus and the final composition obtained for F1 is the following: 2690 µg PU 534 µg Chol/ 45µg HA.

### II.3. In-vivo experiment #1

The virosome preparations (excepted for the commercially available Inflexal V^{®} vaccine) were purified on sucrose gradients as described in Example II.2.1 and II.2.2.
Injected virosomes have the following composition:
**2690** µg PU **534** µg Chol/ **45µg** HA (327)
**6160** µg PL/**1805** µg Chol/ **45**µg HA (328)

Inflexal V^{®} vaccine (commercially available from Berna) composition was +/- 140 µg PU 3 x 15 µg HA.

Animals were bled pre-vaccination (day 63) and 14 days post-vaccination. Hemagglutination inhibiting antibody (HI) responses were measured using standard techniques (Dowdle et al., 1979. Influenza Viruses. In: Diagnostic procedures for viral, rickettsial, and chlamydial infections. American Public Health Association, Washington, D.C. pp 585-609) and geometric mean titers are shown in Figures 3 (A/New Caledonia H1N1) and 4 (A/Panama H3N2).

### Results

The two monovalent preparations were injected separately (hence the response to either A/New Caledonia or A/Panama). For the A/New Caledonia the virosome preparation with or without QS21 induced significantly higher HI (serum haemagglutinin inhibition titres, see example IV) antibody response than the split plain (un-adjuvanted) vaccine, while for the A/Panama the addition of QS21 led to an HI response higher than split plain. Both formulations induced a higher response than that obtained with a virosome-based commercially available vaccine, Inflexal V^{®}, which was generally similar to that induced by split plain vaccine. Thus, the QS21 adjuvanted virosome formulations were able to induce the most potent HI antibody responses of all formulations tested.

### II.4. Plan of experiment #2

In a separate experiment new preparations of Influenza A/H1N1 virosomes were tested for immunogenicity in the primed mouse model. As in the previous experiment the mice were primed on day 0 with whole inactivated trivalent influenza virus (A/New Caledonia H1N1, A/Panama H3N2, and B/Shangdong) by the intranasal route. On day 42 the animals were vaccinated with:
- Group A (trivalent split vaccine control): 1.5 µg per strain (HA) of split trivalent vaccine (see above composition) - so-called 'Plain'
- Group B: monovalent virosome preparation V3 with 1.5 µg HA (A/New Caledonia H1N1) - so called 'purified V3'
- Group C: monovalent virosome preparation V3 with 1.5 µg HA (A/New Caledonia H1N1) + 5 µg QS21 - so called 'purified V3 + QS21'
- Group D: monovalent virosome preparation V4 with 1.5 µg HA (A/New Caledonia H1N1) - so called 'purified V4'
- Group E: monovalent virosome preparation V4 with 1.5 µg HA (A/New Caledonia H1N1) + 5 µg QS21 - so called 'purified V4 + QS21'

The virosomes injected in mice of groups B-E were prepared according to the method provided below (see II.5). Their composition was as follows:
V3: 1850 µg PU 409 µg Chol/ 45µg HA
V4: 1200 µg PU 127 µg Chol/ 45µg

### II.5. Preparation of the virosomes

### II.5.1. Description of V3 preparation:

50 ml of BPL-inactivated whole A/New Caledonia/20/99 virus, containing 526 µg HA/ml and 1615 µg total protein/ml are centrifuged for 1H at 60.000 g at 20°C. The supernatant is discarded. Sarcosyl® at 500 mg/ml in PBS pH 7.4 is added to the pellet to reach a final detergent: protein ratio (w/w) of 10. PBS pH 7.4 is also added to reach a final HA concentration of 1 mg/ml. The pellet is triturated to detach it from the vial wall. The mixture is then shaken (orbital shaking) for 2H at RT. Centrifugation at 100 000 g is performed for 1H at 20°C. The supernatant (so-called split) is kept.

Table 3 herebelow shows yields obtained for HA and Protein after splitting (HA is quantified by SRD and protein by Pierce kit 23225 preceeded by a treatment with Compat-Able^{™} ref: 23215 from Pierce). The HA to protein ratio indicates that a splitting has been obtained.

**Table 3**

| | |
|---|---|
| Protein yield | 80% |
| Ha yield | 95% |
| HA/Protein | 0.39 |

In parallel, DOPC (133 mg) and Cholesterol (33 mg) solubilized in chloroform are mixed in a round bottomed flask and chloroform is evaporated. The dried lipid film is reconstituted with 5.8 ml of the split. PBS pH7.4 (4.2 ml) is also added to obtain a final volume of 10 ml volume and a final HA concentration of 600 µg/ml. The flask is shaken (orbital shaking) for 3H at RT to allow mixed micelles formation. At this step, the final DOPC/ Cholesterol/ HA composition is 1000/250/45. The mixture is then filtered on 0.2µm (Millex- GV; 0.22µm Filter unit; ref: SLGV013SL).

**Figure 7** shows the HA, Phospholipids (PL) and Cholesterol yields. Those are close to 100%.

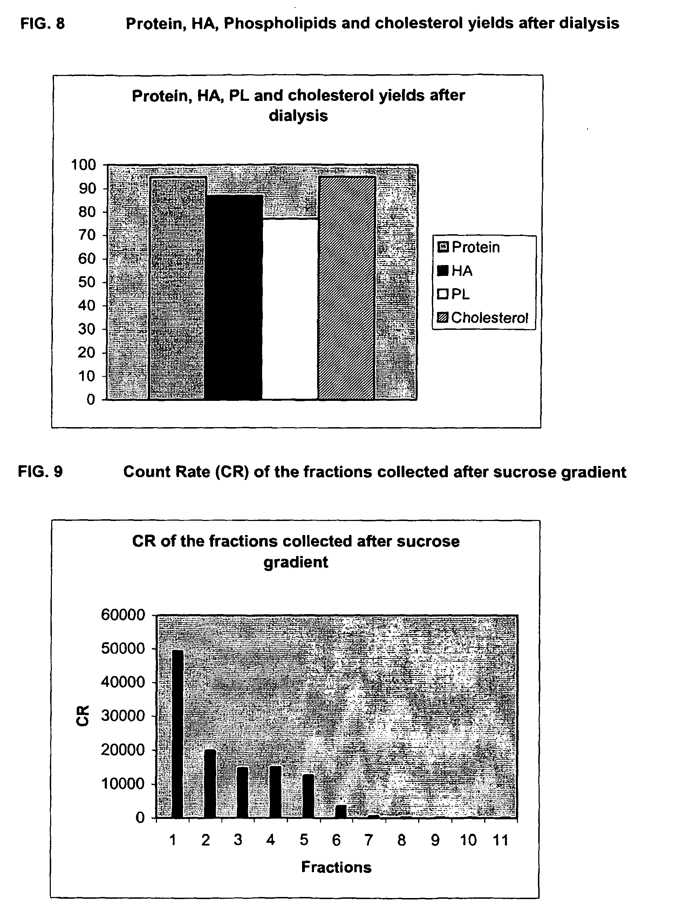

Dialysis in Slide-A-Lyser cassette 3-12 ml capacity (10000 MWCO from Pierce #66810) is then performed against PBS pH 7.4 for 3 days with 3 buffer changes. Protein (Lowry method), HA (by SRD), PL (MPR2 kit) and Cholesterol (Boehringer Mannheim kit) contents are determined. The yields obtained are summarized in Figure 8. A size of 97 nm (as measured with the Zetasizer 3000HS) has been obtained with a polydispersity of 0.2.

A purification step is performed on a 5-45% sucrose gradient: several tubes are prepared (in 14x89 mm Untra Clear tubes) on which 500 µl of the sample are put. Gradients are run for 17H (SW41Ti rotor, 34000 rpm, T° 10°C). Fractions of 1 ml are then collected by the top (F1 is the first fraction on the top of the gradient, F12 is the bottom fraction of the gradient). The Count Rate (CR) being the number of scattered photons, is measured (Figure 9) which indicates where particles are present. Most particles are present in fraction 1 (F1) as well as in fractions F2 to F4. An SDS-Page gel is also performed and this one indicates that HA is also present in those fractions. Fractions F1 to F3 have been pooled together (= Pool F1,2,3 or Viro3 - V3 - purified) and dialyzed against PBS pH 7.4 to eliminate sucrose. HA, Protein, phospholipids and cholesterol are determined.

Global yields (from the whole virus to the Pool F1,2,3) as well as the final composition of the pool F1,2,3 are summarised in Table 4.

**Table 4**

| | Protein | HA | PL | Cholesterol | Final composition: DOPC/ Cholesterol/ HA |
|---|---|---|---|---|---|
| V3 F1,2,3 | 51% | 57% | 113% | 98% | 1850/409/45 |

### II.5.2. Description of V4 preparation:

50ml of BPL-inactivated whole A/New Caledonia/20/99 virus, containing 526 µg HA/ml and 1615 µg total protein/ml are centrifuged for 1H at 60.000 g at 20°C. Supernatant is discarded. Sarcosyl® at 500 mg/ml in PBS pH 7.4 is added to the pellet to reach a final detergent: protein ratio (w/w) of 10. PBS pH 7.4 is also added to reach a final HA concentration of 1 mg/ml. The pellet is triturated to detach it from the vial wall. The mixture is than shaken (orbital shaking) for 2H at RT. Centrifugation at 100 000 g is performed for 1H at 20°C. The supernatant (also called split) is kept.

Table 5 herebelow shows the yields obtained for HA and Protein after splitting (HA quantified by SRD and protein by Pierce kit 23225 preceded by a treatment with Compat-Able^{™} ref: 23215 from Pierce). The HA to protein ratio indicates that a splitting has been obtained.

**Table 5**

| | |
|---|---|
| Protein yield | 80% |
| Ha yield | 95% |
| HA/Protein | 0.39 |

In parallel, DOPC (66.6 mg), Cholesterol (16.6 mg) and Sarcosyl® (66.6 mg), solubilized in chloroform are mixed in a round bottomed flask and chloroform is evaporated. The dried lipid film is reconstituted with 5.8 ml of the split preparation. PBS pH7.4 (4.2ml) is also added to obtain a final volume of 10 ml volume and a final HA concentration of 600 µg/ml. The flask is shaken (orbital shaking) for 3H at RT to allow mixed micelles formation. At this step, the final DOPC/ Cholesterol/ HA composition is 500/125/45.

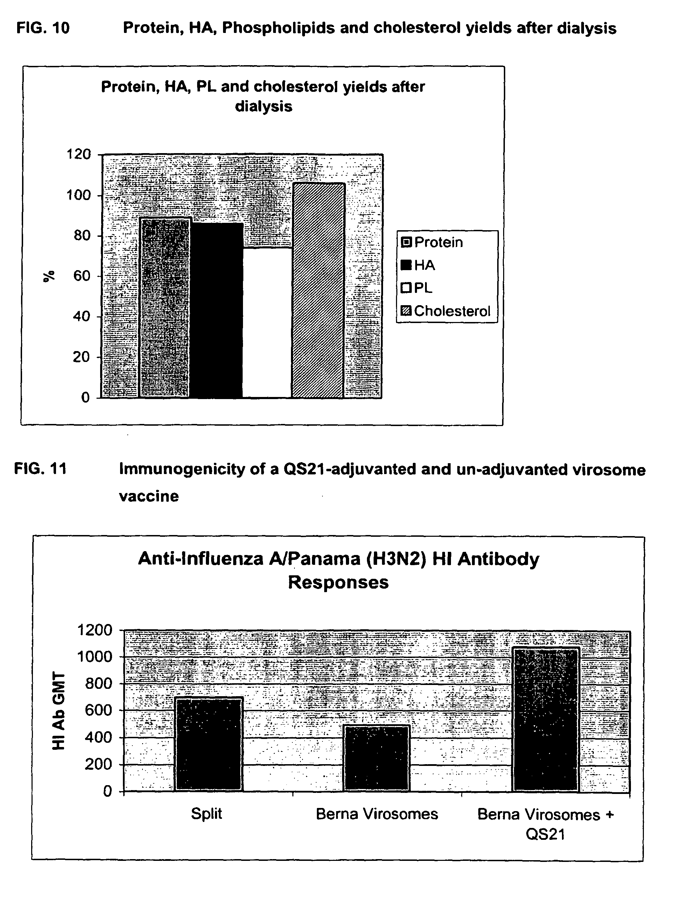

The mixture is then filtered on 0.2 µm (Millex- GV; 0.22µm Filter unit; ref: SLGV013SL). Yields after filtration are close to 100% (HA, Protein, PL, Cholesterol). Dialysis in Slide-A-Lyser cassette 3-12 ml capacity (10000MWCO from Pierce #66810) is then performed against PBS pH 7.4 for 3 days with 3 buffer changes. Protein (Lowry method), HA (by SRD), PL (MPR2 kit) and Cholesterol (Boehringer Mannheim kit) contents are determined. The yields obtained are summarized in Figure 10. A size (measured with the Zetasizer 3000HS) of 88 nm was obtained with a polydispersity of 0.16.

A purification step is performed on 5-45% sucrose gradient: several tubes are prepared (in 14x89 mm Untra Clear tubes) on which 500 µl of the sample are layered. Gradients are run for 17H (SW41Ti rotor, 34000rpm, T° 10°C). Fractions of 1 ml are collected from the top (F1 is the first fraction on top of the gradient, F12 is at the bottom of the gradient). The Count Rate (CR) (measured with the Zetasizer3000HS) is measured which indicates that particles are present in fractions F1 to F4. An SDS-Page gel is also performed which indicates that HA is also present in those fractions.

Fractions 1 to 4 are pooled together (=Viro4 - V4 - purified) and dialyzed against PBS pH 7.4 to eliminate sucrose. HA, phospholipids and cholesterol are determined. Global yields (from the whole virus to the Viro4 purified) as well as the final composition of the Viro4 purified are summarized in Table 6.

**Table 6**

| | Protein | HA | PL | Cholesterol | Final composition: DOPC/ Cholesterol/ HA |
|---|---|---|---|---|---|
| V4 Purified | 41% | 70% | 146% | 46% | 1200/127/45 |

The purified virosomes are adjuvanted with QS21 (45 µg HA, 50 µg QS21 meaning 127 µg cholesterol in 500 µl final volume). The size of the final formulation is 79 nm.

### II.5.3. Formulation prior to administration to mice:

Water for injection and PBS 10 times concentrated (will lead to PBS pH 7.4 after 10 times dilution) are mixed together at RT. HA 15 µg in virosomes is added and mixed gently for 10 minutes. QS21 50 µg in H2O is then added and the formulation is gently shaken for 15 minutes.

V3 virosomes final composition is: 15µg HA, 50µg QS21, 136µg Cholesterol and 617µg DOPC per 500µl. The size measured for the final formulation (Zetasizer 3000HS) gives a z average mean of 88nm with a polydispersity equal to 0.16. This size is stable after 7 days storage at 4°C.

### II.6. In vivo experiment #2

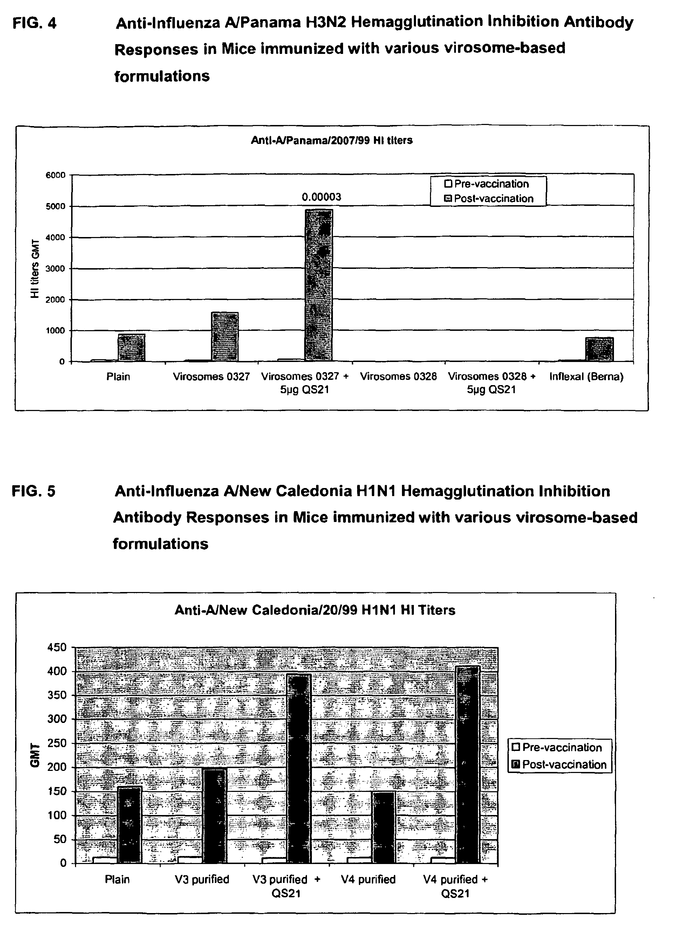

Animals were bled pre-vaccination (day 42) and 14 days post-vaccination (D56). Hemagglutination inhibiting antibody (HI) responses were measured using standard techniques (Dowdle et al., 1979. Influenza Viruses. In: Diagnostic procedures for viral, rickettsial, and chlamydial infections. American Public Health Association, Washington, D.C. pp 585-609) and geometric mean titers are shown in Figure 5 (Anti-A/New Caledonia H1N1).

In this experiment the immunogenicity of both the H1N1 virosome preparations was generally similar to that of the split plain vaccine, while addition of QS21 to the formulations resulted in at least a 2-fold boost in the antibody responses to the virosome-based formulations. While the immunogenicity was increased by the addition of QS21 adjuvant there was no difference in the reactogenicity profile observed during the experiment. In conclusion, the novel QS21-adjuvanted virosome formulations provide an advantage in terms of immunogenicity of influenza vaccines administered by the parenteral route while maintaining an acceptable reactogenicity profile.

### Example III - SRD Method used to measure haemagglutinin (HA) content (Illustrative example)

Glass plates (12.4 - 10.0 cm) are coated with an agarose gel containing a concentration of anti-influenza HA serum that is recommended by NIBSC. After the gel has set, 72 sample wells (3 mm Ø) are punched into the agarose. 10 microliters of appropriate dilutions of the reference and the sample are loaded in the wells. The plates are incubated for 24 hours at room temperature (20 to 25°C) in a moist chamber. After that, the plates are soaked overnight with NaCl-solution and washed briefly in distilled water. The gel is then pressed and dried. When completely dry, the plates are stained on Coomassie Brillant Blue solution for 10 min and destained twice in a mixture of methanol and acetic acid until clearly defined stained zones become visible. After drying the plates, the diameter of the stained zones surrounding antigen wells is measured in two directions at right angles. Alternatively equipment to measure the surface can be used. Dose-response curves of antigen dilutions against the surface are constructed and the results are calculated according to standard slope-ratio assay methods (Finney, D.J. (1952). Statistical Methods in Biological Assay. London: Griffin, Quoted in: Wood, JM, et al (1977). J. Biol. Standard. 5, 237-247).

### Example IV - Haemagglutination Inhibition (HAI) activity of influenza-specific serum antibodies (Illustrative example)

Sera (50 µl) are treated with 200 µl RDE (receptor destroying enzyme) for 16 hours at 37°C. The reaction is stopped with 150 µl 2.5% Na citrate and the sera are inactivated at 56°C for 30 min. A dilution 1:10 is prepared by adding 100 µl PBS. Then, a 2-fold dilution series is prepared in 96 well plates (V-bottom) by diluting 25 µl serum (1:10) with 25 µl PBS. 25 µl of the reference antigens are added to each well at a concentration of 4 hemagglutinating units per 25 µl. Antigen and antiserum dilution are mixed using a microtiter plate shaker and incubated for 60 minutes at room temperature. 50 µl chicken red blood cells (RBC) (0.5%) are then added and the RBCs are allowed to sediment for 1 hour at RT. The HAI titre corresponds to the inverse of the last serum dilution that completely inhibits the virus-induced hemagglutination.

### Example V - Comparative immunogenicity experiment using three influenza vaccine preparations

In this in vivo experiment, the immunogenicity of a classical split vaccine was compared to the commercially available Inflexal V® vaccine (Berna 2001-2002 composition with A/New Caledonia/20/99 like, A/Moscow/10/99 like, B/Sichuan/379/99 like strains) and to the Inflexal V® vaccine adjuvanted with a detoxified (i.e. quenched) form of QS21 (so-called DQ, see WO 96/33739, herein incorporated by reference) are compared.

In this immunogenicity experiment mice were again primed by the intranasal route on day 0 with 5 µg of trivalent whole inactivated influenza virus (A/New Caledonia/20/99 H1N1, A/Panama/2007/99 H3N2, and B/Johannesburg/5/99). On day 28 the animals were vaccinated by the IM route with classical split vaccine (1.5 µg HA content per strain), the Inflexal V (Berna) virosome vaccine (1.5 µg HA content per strain), or the Inflexal V (Berna) virosome vaccine (1.5 µg HA content per strain) adjuvanted with 5 µg detoxified QS21. The Inflexal V® - QS21 formulation is prepared as follows: to 500 µl of Inflexal V® vaccine containing 15 µg HA per strain (total HA content of 45µg), 50 µg of QS21 in its DQ form is added (DQ: 50 µg QS21, 250 µg Cholesterol, 1000 µg DOPC).

The HI antibody response (determined according to the method of Example IV) was measured 14 days after vaccination and the results seen for the A/Panama strain are shown in Figure 11. Briefly the HI antibody titers induced by vaccination were generally similar for the split and the un-adjuvanted Berna virosome vaccine. However, upon addition of detoxified QS21 the HI antibody titer increased by approximately 2-fold. Similar results were obtained with the other two strains.

This experiment has been repeated and the same results were obtained.

### Example VI - Lytic activity of commercially available virosomes Inflexal V® adjuvanted with a detoxified form of QS21

Inflexal V® 2001/2002 composition (with A/ New Caledonia/20/99 like, A/Moscow/10/99 like, B/Sichuan/379/99 like strains) was used (commercially available from Berna), which contains 15 µg of each strain in 500 µl final.

Two detoxified forms of QS21 have been used in this experiment called DQ or AS01 (as disclosed in WO96/33739). DQ or AS01 were added to the virosome preparation so as to reach a QS21 amount of approximately 100 µg/ml or 20 µg/ml (i.e. approximately 50 or 10 µg per 0.5 ml dose). Table 7 summarises the formulations prepared.

**Table 7**

| Group | Inflexal V® | DQ | AS01 | PBS pH 7.4 | Final volume | Equivalent of QS21 |
|---|---|---|---|---|---|---|
| 1. Inflexal V® + DQ/ 5 | 500 µl | 50 µl | - | - | 550 µl | 50 µg |
| 2. Inflexal V® + AS01/ 5 | 500 µl | - | 50 µl | - | 550 µl | 50 µg |
| 3. Inflexal V® + DQ/ 1 | 500 µl | 10 µl | - | 40 µl | 550 µl | 10 µg |
| 4. Inflexal V® + AS01/1 | 500 µl | - | 10 µl | 40 µl | 550 µl | 10 µg |
| 5. Inflexal V® control | 500 µl | - | - | 50 µl | 550 µl | 0 µg |

Groups 1 and 2 have a final QS21 concentration of 91 µg/ml (50µg/550µl) Groups 3 and 4 a concentration of 18.2µg/ml (10µg/550µl).

The lytic activity associated with QS21 is tested against red blood cells and lysis, should it occur, is measured by OD at 540nm, according to the procedure detailed below:
Red blood cells are washed 3 times by low speed centrifugation of the blood followed by resuspension of the red cells in PBS to the original volume. After washing, the red cells pellet is diluted 10 times in PBS.
QS21 references are prepared: 1, 2.5 and 5 µg of QS21 are mixed with PBS to reach a final volume of 900 µl. For the samples to be tested a volume containing the equivalent of about 2 µg of QS21 is taken and PBS is added to reach a volume of 900 µl.
100 µl of red blood cells (diluted 10 times) are added to each reference and sample. The tubes are shaken very gently and let for 30 minutes at room temperature (RT). The tubes are then centrifuged at 2000 rpm (CS-6R centrifuge- Beckman) for 5 minutes.

The OD of the supernatant is read at 540nm.
Results are illustrated in Table 8

**Table 8**

| Sample | µg QS21 | OD |
|---|---|---|
| Blank | 0 | 0.086 |
| QS21 | 1 µg | 0.217 |
| QS21 | 1.5 µg | 0.631 |
| QS21 | 2.5 µg | 0.891 |
| Inflexal V® + DQ/ 5 | 2.3 µg | 0.083 |
| Inflexal V® + AS01/ 5 | 2.3 µg | 0.078 |
| Inflexal V® + DQ/ 1 | 1.46 µg | 0.080 |
| Inflexal V® + AS01/ 1 | 1.46 µg | 0.079 |
| Inflexal V® control | 0 µg | 0.080 |

As observed in Table 8, the OD obtained for the samples containing the detoxified form of QS21 is equivalent to the OD obtained for the blank (PBS control). This means that no lytic activity can be detected. QS21 remains detoxified in those formulations.

## Claims

1. A composition comprising an influenza virosome preparation and QS21.

2. A composition as claimed in claim 1 additionally comprising an exogeneous sterol.

3. A composition according to claim 2 wherein the exogeneous sterol is selected from the list consisting of: sitosterol, stigmasterol, ergosterol, ergocalciferol and cholesterol.

4. A composition according to claim 2 or claim 3, wherein QS21 is formulated with the sterol to form a liposomal structure.

5. A composition according to claim 2 or claim 3, wherein QS21 is formulated with the sterol to form an ISCOM structure.

6. A composition according to any of claims 1 to 5, wherein the virosome preparation further comprises an exogeneous phospholipid.

7. A composition according to claim 6, wherein the exogeneous phospholipid is phosphatidylcholine or a derivative thereof.

8. An composition according to any of claims 1 to 7, further comprising an additional adjuvant.

9. A composition as claimed in claim 8 wherein the additional adjuvant is selected from the group consisting of: a Toll like receptors agonist, aluminium hydroxide, aluminium phosphate, tocopherol, and an oil-in-water emulsion or a combination of two or more of the said adjuvants.

10. A composition as claimed in claim 9 wherein the Toll like receptors agonist is selected from the group consisting of: in particular Toll like receptor 2 agonist, Toll like receptor 3 agonist, Toll like receptor 4 agonist, Toll like receptor 7 agonist, Toll like receptor 8 agonist and Toll like receptor 9 agonist.

11. A method for preparing an immunogenic composition, comprising combining an influenza virosome preparation with QS21.

12. A method as claimed in claim 11, wherein the influenza virosome preparation is as claimed in any of claims 1 to 10.

13. A composition according to any of claims 1 to 10 for use in medicine.

14. The use of an influenza virosome preparation, and of QS21 in the manufacture of a medicament for the prophylaxis of an infection or disease caused by said virus in an individual susceptible to it.

15. Use as claimed in claim 14, wherein the individual is an elderly patient

## Patentansprüche

1. Zusammensetzung, umfassend eine Influenza-Virosomen-Zubereitung und QS21.

2. Zusammensetzung gemäss Anspruch 1, die zusätzlich ein exogenes Sterin umfasst.

3. Zusammensetzung gemäss Anspruch 2, wobei das exogene Sterin aus der Liste bestehend aus Sitosterin, Stigmasterin, Ergosterin, Ergocalciferol und Cholesterin ausgewählt ist.

4. Zusammensetzung gemäss Anspruch 2 oder Anspruch 3, wobei QS21 mit dem Sterin zur Bildung einer liposomalen Struktur formuliert ist.

5. Zusammensetzung gemäss Anspruch 2 oder Anspruch 3, wobei QS21 mit dem Sterin zur Bildung einer ISCOM-Struktur formuliert ist.

6. Zusammensetzung gemäss einem der Ansprüche 1 bis 5, wobei die Virosomen-Zubereitung ferner ein exogenes Phospholipid umfasst.

7. Zusammensetzung gemäss Anspruch 6, wobei das exogene Phospholipid Phosphatidylcholin oder ein Derivat davon ist.

8. Zusammensetzung gemäss einem der Ansprüche 1 bis 7, die ferner ein zusätzliches Adjuvans umfasst.

9. Zusammensetzung gemäss Anspruch 8, wobei das zusätzliche Adjuvans aus der Gruppe bestehend aus einem Agonisten der Toll-ähnlichen Rezeptoren, Aluminiumhydroxid, Aluminiumphosphat, Tocopherol und einer Öl-in-Wasser-Emulsion oder einer Kombination von zwei oder mehreren der Adjuvantien ausgewählt ist.

10. Zusammensetzung gemäss Anspruch 9, wobei der Agonist der Toll-ähnlichen Rezeptoren aus der Gruppe bestehend aus einem Agonisten des Toll-ähnlichen Rezeptors 2, einem Agonisten des Toll-ähnlichen Rezeptors 3, einem Agonisten des Toll-ähnlichen Rezeptors 4, einem Agonisten des Toll-ähnlichen Rezeptors 7, einem Agonisten des Toll-ähnlichen Rezeptors 8 und einem Agonisten des Toll-ähnlichen Rezeptors 9 ausgewählt ist.

11. Verfahren zur Herstellung einer immunogenen Zusammensetzung, umfassend das Kombinieren einer Influenza-Virosomen-Zubereitung mit QS21.

12. Verfahren gemäss Anspruch 11, wobei die Influenza-Virosomen-Zubereitung wie in einem der Ansprüche 1 bis 10 beansprucht ist.

13. Zusammensetzung gemäss einem der Ansprüche 1 bis 10 zur Verwendung in der Medizin.

14. Verwendung einer Influenza-Virosomen-Zubereitung und QS21 bei der Herstellung eines Medikaments zur Vorbeugung einer Infektion oder Erkrankung, die durch den Virus verursacht wird, in einem Individuum, das für diesen empfänglich ist.

15. Verwendung gemäss Anspruch 14, wobei das Individuum ein älterer Patient ist.

## Revendications

1. Composition comprenant une préparation de virosome d'influenza et de QS21.

2. Composition selon la revendication 1, comprenant en outre un stérol exogène.

3. Composition selon la revendication 2, dans laquelle le stérol exogène est choisi dans la liste comprenant le silostérol, le stigmastérol, l'ergostérol, l'ergocalciférol et le cholestérol.

4. Composition selon la revendication 2 ou la revendication 3, dans laquelle QS21 est formulé avec le stérol pour former une structure liposomale.

5. Composition selon la revendication 2 ou la revendication 3, dans laquelle QS21 est formulée avec le stérol pour former une structure ISCOM.

6. Composition selon l'une quelconque des revendications 1 à 5, dans laquelle la préparation de virosome comprend en outre un phopholipide exogène.

7. Composition selon la revendication 6, dans laquelle le phospholipide exogène est la phophatidylcholine ou un dérivé de celle-ci.

8. Composition selon l'une quelconque des revendications 1 à 7, comprenant en outre un adjuvant supplémentaire.

9. Composition selon la revendication 8, dans laquelle l'adjuvant supplémentaire est choisi dans le groupe comprenant un agoniste des récepteurs de type Toll, l'hydroxyde d'aluminium, le phosphate d'aluminium, le tocophérol et une émulsion huile dans eau ou une combinaison de deux ou plusieurs desdits adjuvants.

10. Composition selon la revendication 9, dans laquelle l'agoniste des récepteurs de type Toll est choisi dans le groupe comprenant en particulier, l'agoniste du récepteur 2 de type Toll, l'agoniste du récepteur 3 de type Toll, l'agoniste du récepteur 4 de type Toll, l'agoniste du récepteur 7 de type Toll, l'agoniste du récepteur 8 de type Toll et l'agoniste du récepteur 9 de type Toll.

11. Procédé de préparation d'une composition immunogène, comprenant la combinaison d'une préparation de virosome d'influenza avec QS21.

12. Procédé selon la revendication 11, dans lequel la préparation du virosome d'influenza est selon l'une quelconque des revendications 1 à 10.

13. Composition selon l'une quelconque des revendications 1 à 10, pour l'utilisation en médecine.

14. Utilisation d'une préparation de virosome d'influenza et de QS21 dans la production d'un médicament pour la prophylaxie d'une infection ou d'une maladie provoquée par ledit virus chez un individu sensible à celui-ci.

15. Utilisation selon la revendication 14, dans laquelle l'individu est un patient âgé.
